# FASCICULE DE BREVET EUROPEEN

(11) **EP 2 482 853 B1**
(45) Date de publication et mention de la délivrance du brevet: **14.11.2018**
(21) Numéro de dépôt: 10762659.0
(22) Date de dépôt: 04.10.2010
(51) Int. Cl.: A61K 31/5415, A61K 47/26, A61K 47/54

(54) **TRANSPORT COOPERATIF DE PRINCIPES ACTIFS BASIQUES PAR DES MOLECULES AMPHIPHILES ACIDES**
KOOPERATIVE ÜBERMITTLUNG VON BASISCHEN WIRKSTOFFEN DURCH AMPHIPHILE SÄUREMOLEKÜLE
COOPERATIVE CONVEYANCE OF BASIC ACTIVE PRINCIPLES BY AMPHIPHILIC ACID MOLECULES

(30) Priorité: 02.10.2009 FR 0956893
(43) Date de publication de la demande: 08.08.2012
(73) Titulaire: Centre National de la Recherche Scientifique (C.N.R.S.), 75016 Paris (FR); Université Paul Sabatier Toulouse III, 31400 Toulouse (FR)
(72) Inventeur: BIZE, Cécile, F-31500 Toulouse (FR); BLANZAT, Muriel, F-31400 Toulouse (FR); PEREZ, Emile, F-31770 Colomiers (FR); RICO-LATTES, Isabelle, F-31650 Auzielle (FR)
(74) Mandataire: Lavoix
(86) Numéro de dépôt international: PCT/EP2010/064756
(87) Numéro de publication internationale: WO 2011/039379

(56) Documents cités:
- FR-A1- 2 727 110
- BIZE C ET AL: "Physicochemical studies of the supramolecular organization of catanionic bolaamphiphilic associations", COMMUNICACIONES PRESENTADAS A LAS JORNADAS DE COMITE ESPAGNOLDE LA DETERGENCIA, BARCELONA, ES, vol. 38, 1 janvier 2008 (2008-01-01), pages 225-234, XP008122612, ISSN: 0212-7466

## Description

La présente invention concerne une association catanionique intermoléculaire d'un principe actif basique et d'un tensioactif acide permettant de protéger, solubiliser et véhiculer le principe actif.

L'article de BIZE C et Al. "Physicochemical studies of the supramolecular organization of catanionic bolaamphiphilic associations", Communicaciones presentadas a las jornadas de comitE espagnolde de la detergencia, Barcelona, ES, Vol. 38, 2008, pages 225-234 divulgue des complexes d'association intermoléculaire d'un transporteur amphiphile et d'un composé comprenant une fonction amine.

Améliorer la stabilité d'un principe actif afin de réduire ses effets secondaires reste encore de nos jours un enjeu très important. Généralement, cette stabilisation se réalise à la fois lors du stockage du principe actif par l'utilisation de conservateurs et d'anti-oxydants, et lors de l'administration du principe actif par l'utilisation d'un véhicule permettant de solubiliser celui-ci et éventuellement de le protéger des dégradations dues au système immunitaire et de le véhiculer vers son site d'action.

De nos jours, il existe de nombreuses techniques de vectorisation de principes actifs. Ces techniques utilisent différentes stratégies en fonction de la nature du principe actif (hydrophile ou lipophile), ainsi que de l'organe visé, de la dose administrée et de la durée d'administration. Par exemple, le principe actif pourra être encapsulé à l'intérieur d'une vésicule de phospholipides ou immobilisé dans des microsphères de polymère biodégradable.

Par ailleurs, la délivrance de principes actifs au travers de la peau présente de nombreux avantages. Les vitesses variables d'absorption et de métabolisation liées à un traitement par voie orale sont évitées, ainsi que d'éventuelles irritations gastro-intestinales. La délivrance du principe actif par voie transcutanée permet également de mieux contrôler sa concentration sanguine.

Toutefois, la peau présente une structure complexe et les molécules administrées par voie transcutanée ou topique doivent tout d'abord franchir une première barrière constituée par le *stratum corneum* avant d'atteindre le courant sanguin. Le *stratum corneum* est constitué d'une couche dense et hautement kératinisée d'une épaisseur moyenne de 10-15 microns. Le degré élevé de kératinisation, ainsi que l'assemblage compact des cellules peut constituer une barrière pratiquement imperméable au passage d'un principe actif.

Pour la plupart des médicaments, la vitesse de perméabilisation au travers de la peau, sans adjonction d'additif perméabilisant, est extrêmement lente. L'efficacité de l'administration cutanée repose donc essentiellement sur le système de délivrance utilisé qui conditionne la stabilité du principe actif ainsi que sa biodisponibilité et sa biositribution cutanée. Les stratégies adoptées pour faire pénétrer une quantité suffisante de principe actif impliquent donc généralement l'utilisation de promoteurs de pénétration ou des formulations sophistiquées.

De nombreux additifs peuvent ainsi être utilisés afin d'augmenter la vitesse de pénétration du principe actif au travers de la peau. La plupart des composés sont administrés en même temps que le médicament (dans certains cas la peau peut être prétraitée avec un agent de perméabilisation) de manière à augmenter la perméabilité du *stratum corneum* et ainsi accroître la pénétration du principe actif au travers de la peau. La perméabilité de beaucoup d'agents thérapeutiques peut être améliorée grâce à ces agents de perméabilisation.

Plusieurs additifs sont capables de promouvoir le transport de principes actifs au travers de la peau selon différents mécanismes dont les plus importants sont les suivants :
- Extraction des lipides du *stratum corneum*
- Désorganisation de la structure de la bicouche lipidique
- Déplacement de l'eau liée
- Délamination du *stratum corneum*
- Désorganisation de la couche cornée

Les agents de perméabilisation peuvent être classés dans différentes catégories. Ainsi, des solvants tels que des alcools, des alkyle méthyle sulfoxydes et des polyols, augmentent la solubilité ce qui accroît le passage cutané. Quelques solvants comme le diméthylsulfoxyde (DMSO) ou l'éthanol, pourront extraire les lipides et rendre le *stratum corneum* plus perméable. L'acide oléique et le myristate d'isopropyle sont des exemples types d'agents de perméabilisation qui désorganisent la couche cornée en s'intercalant dans les structures lipidiques. Cet effet émollient accroît ainsi le coefficient de diffusion du principe actif. Egalement, des tensioactifs ioniques ou le DMSO interagissent avec la kératine des cornéocytes, ce qui déploie la structure de la protéine et augmente le coefficient de diffusion.

Cependant, la plupart de ces systèmes de délivrance présente l'inconvénient majeur d'induire des altérations irréversibles au niveau de la peau.

La présente invention décrit une stratégie originale qui consiste à faire participer le principe actif lui-même de manière active à son propre transport, permettant de protéger, solubiliser et véhiculer jusqu'à son site d'action le médicament. Pour cela, nous proposons d'associer par simple interaction électrostatique acide/base, un principe actif basique avec une molécule amphiphile acide bio compatible, cette association pouvant être stabilisée en outre par des interactions de type hydrophobes entre le principe actif et la molécule amphiphile.

En particulier, cette invention présente des applications en formulation, en tant que solubilisation, protection, transport et diffusion transcutanée d'un principe actif, la molécule amphiphile pouvant jouer également le rôle d'agent de perméabilisation pour un transport transcutané. Ces formulations simplifiées et inoffensives pour la peau permettent alors d'augmenter la concentration en principe actif au niveau de la peau et de favoriser son passage à travers le *stratum corneum.*

L'invention propose ainsi d'associer un transporteur amphiphile acide biocompatible à un principe actif comportant une ou plusieurs fonctions basiques. Cette association intermoléculaire conduit à une nouvelle espèce amphiphile correspondant à la formation d'une paire acide/base liée par des interactions électrostatiques et stabilisée par des interactions de type Van der Waals entre les parties hydrophobes des deux constituants. Le complexe amphiphile ainsi formé par association, pourra conduire en fonction de sa concentration dans l'eau ainsi que de la nature du principe actif (volume, hydrophobie), à un ensemble de structures auto-assemblées telles que des micelles ou des vésicules. Les objets ainsi formées pourront également servir à l'auto-transport du principe actif.

La présente invention a donc pour objet un complexe d'association intermoléculaire d'un transporteur amphiphile et d'un principe actif, caractérisé en ce que ledit complexe est choisi parmi :

| | |
|---|---|
| **Lac6P** | 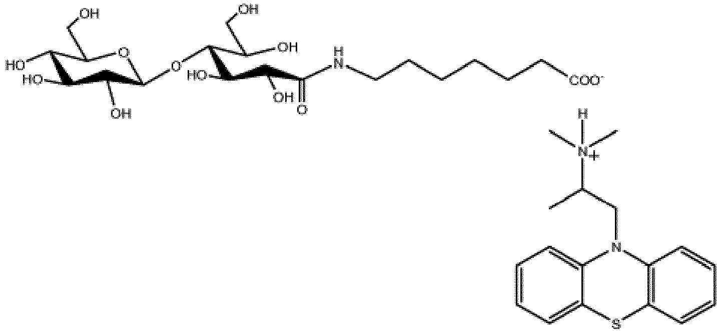 |
| **Glu6P** | 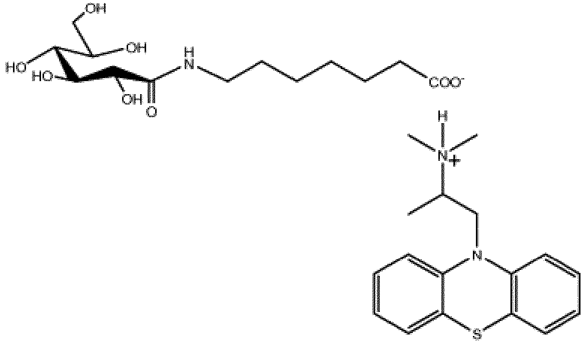 |

| | |
|---|---|
| **Lac6M** | 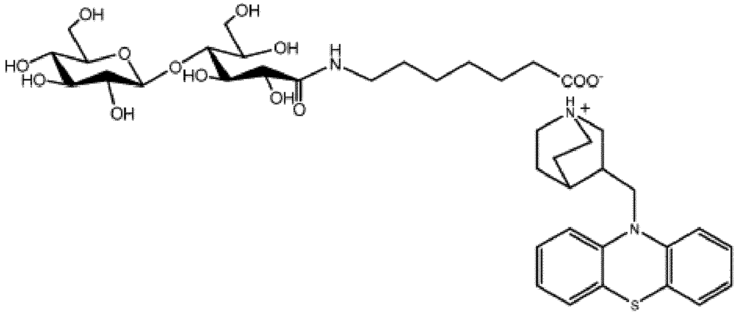 |
| **Glu6M** | 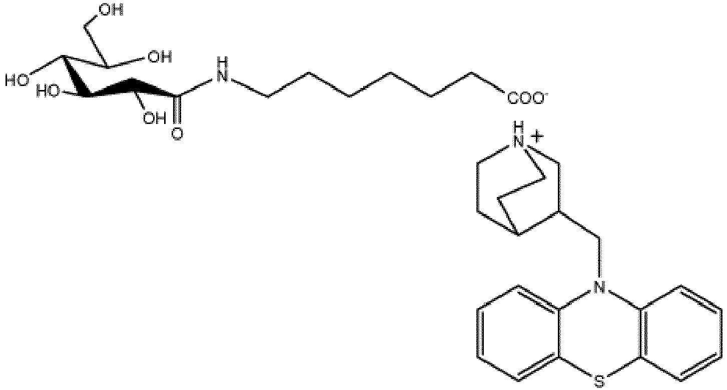 |

Il est également décrit un complexe d'association intermoléculaire d'un transporteur amphiphile et d'un principe actif de formule générales (I) suivante : 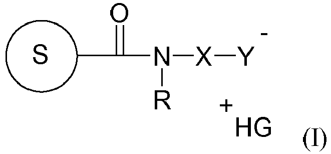 dans laquelle :
- S représente un fragment d'un glucide, tel qu'un monosaccharide ou un polysaccharide.
- R représente un atome d'hydrogène, une chaîne hydrocarbonée en C₁-C₂₀, avantageusement en C₁-C₁₀, saturée ou insaturée, linéaire, ramifiée, cyclique ou aromatique, éventuellement substituée par un ou plusieurs atomes de fluor, notamment perfluorée,
- X représente une chaîne alkyle, alcényle ou alcynyle en C₄ à C₁₀, de préférence linéaire,
- Y⁻ représente un groupe carboxylate (-CO₂⁻), sulfate (-O-SO₃⁻), sulfonate(-SO₃⁻), phosphate (-O-P(O)(ORₐ)O⁻), phosphonate (-O-P(O)R_{b}O⁻) ou phosphinate (-P(O)R_{b}O⁻),
   avec Rₐ représentant un groupe alkyle en C₁ à C₆ et R_{b} représentant un atome d'hydrogène ou un groupe alkyle en C₁ à C₆, et
- G représente un principe actif comprenant au moins une fonction amine primaire, secondaire ou tertiaire ou guanidine (-NH-C(=NH)-NH₂), notamment choisi dans le groupe comprenant les antihistaminiques, les antidépresseurs, les hypotenseurs, les neurotransmetteurs, les amphétamines, les anesthésiants, les antimigraineux, les stimulants cardiaques, les agonistes des récepteurs nicotiniques, les vitamines ou pro-vitamines, et les anticancéreux.

Dans le cadre de la présente invention, les termes suivants seront utilisés indifféremment pour désigner les complexes d'association intermoléculaire d'un transporteur amphiphile et d'un principe actif selon l'invention : « complexe », « complexe d'association intermoléculaire », « complexe catanionique », ou encore « association catanionique ».

Par « glucide », on entend, au sens de la présente invention, un monosaccharide ou un polysaccharide.

Par « monosaccharide », on entend, au sens de la présente invention, un aldose, c'est-à-dire un sucre portant une fonction aldéhyde (-CHO). Il peut s'agir notamment de l'érythrose, du thréose, du ribose, de l'arabinose, du xylose, du lyxose, de l'allose, de l'altrose, du glucose, du mannose, du gulose, de l'idose, du galactose, ou du talose, sous une forme D ou L. Il s'agit en particulier du glucose.

Par « polysaccharide », on entend, au sens de la présente invention, un enchaînement d'au moins deux motifs monosaccharide tel que défini ci-dessus. Il pourra s'agir en particulier d'un disaccharide (enchaînement de deux motifs monosaccharide), tel que le lactose.

Par « fragment d'un glucide », on entend, au sens de la présente invention, le fragment du glucide dépourvu de sa fonction aldéhyde.

Par « alkyle », on entend, au sens de la présente invention, une chaîne hydrocarbonée saturée, linéaire ou ramifiée.

Lorsque cette chaîne alkyle représente la chaîne X, elle comportera 4 à 10, de préférence 6, atomes de carbone et sera avantageusement linéaire. A titre d'exemple, on peut citer une chaîne n-hexyle.

Lorsque cette chaîne alkyle représente un groupe Rₐ ou R_{b}, elle comportera 1 à 6 atomes de carbone et pourra être notamment un groupe méthyle, éthyle, propyle, isopropyle, butyle, isobutyle, sec-butyle, tert-buteyle, pentyle ou encore hexyle.

Par « alcényle », on entend, au sens de la présente invention, une chaîne hydrocarbonée, linéaire ou ramifiée, de préférence linéaire, comportant au moins une double liaison et comportant 4 à 10, de préférence 6, atomes de carbone.

Par « alcynyle », on entend, au sens de la présente invention, une chaîne hydrocarbonée, linéaire ou ramifiée, de préférence linéaire, comportant au moins une triple liaison et comportant 4 à 10, de préférence 6, atomes de carbone.

Par « insaturé », on entend, au sens de la présente invention, que la chaîne hydrocarbonée peut comporter une ou plusieurs insaturations.

Par « insaturation », on entend, au sens de la présente invention, une liaison double ou triple entre deux atomes de carbone.

Par « chaîne hydrocarbonée cyclique », on entend, au sens de la présente invention, un groupement hydrocarboné cyclique, comprenant un ou plusieurs cycles accolés, notamment 1 ou 2 cycles. Cette chaîne pourra être saturée ou insaturée et comprendra de 3 à 20, de préférence de 3 à 10, atomes de carbone. Il pourra s'agir d'un groupement cyclopropyle, cyclopentyle ou encore cyclohexyle.

Par « chaîne hydrocarbonée aromatique », on entend, au sens de la présente invention, un groupement aromatique comportant de 6 à 20, de préférence de 6 à 10, atomes de carbone, et comprenant un ou plusieurs cycles accolés, comme par exemple un groupement phényle ou naphtyle. Avantageusement, il s'agit du phényle.

Par « perfluoré », on entend, au sens de la présente invention, que tous les atomes d'hydrogène de la chaîne hydrocarbonée ont été remplacés par des atomes de fluor.

Ces complexes catanioniques ont la particularité de s'auto-assembler spontanément en assemblages supramoléculaires, tels que des vésicules ou des micelles, qui sont stables thermodynamiquement, ce qui permet d'encapsuler le principe actif et donc de le protéger, et de solubiliser l'ensemble grâce au groupement S hydrophile.

Le transporteur utilisé dans le complexe d'association catanionique selon l'invention est choisi parmi des molécules amphiphiles biocompatibles présentant une ou plusieurs fonctions acides.

Selon la présente invention, le transporteur amphiphile sera choisi parmi les dérivés de glucide présentant une ou plusieurs chaînes hydrophobes, ainsi qu'une ou plusieurs fonctions acides susceptibles d'interagir de manière électrostatique avec le principe actif basique.

Ce transporteur amphiphile répond à la formule générale (II) suivante : 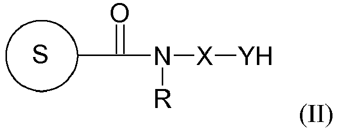 avec S, R, X et Y tels que définis précédemment. Il se trouve alors sous une forme anionique dans le complexe d'association.

S pourra plus particulièrement représenter un fragment d'un glucide, tel qu'un fragment du glucose ou du lactose.

R représentera notamment un atome d'hydrogène ou une chaîne alkyle en C1 à C6, et de préférence représentera un atome d'hydrogène.

Avantageusement, X représentera une chaîne alkyle, alcényle ou alcynyle linéaire en C4 à C10, avantageusement en C5, C6, C7, C8 OU C9, et encore plus avantageusement en C6. Plus particulièrement, X pourra représenter une chaîne alkyle, de préférence linéaire, en C4 à C10, avantageusement en C5, C6, C7, C8 OU C9, et encore plus avantageusement en C6. X pourra donc représenter une chaîne n-hexyle.

Y- représentera en particulier un groupement CO2-.

Le transporteur sera donc avantageusement choisi parmi les tensioactifs acides à tête sucre et à longue chaîne, tels que les acides 1,7-glyconamidoheptanoïques comportant un résidu dérivé de l'acide gluconique ou de l'acide lactobionique que l'on dénommera respectivement Glu6 et Lac6.

La formule générale des acides 1,7-glyconamidoheptanoïques Glu6 et Lac6 est la suivante : 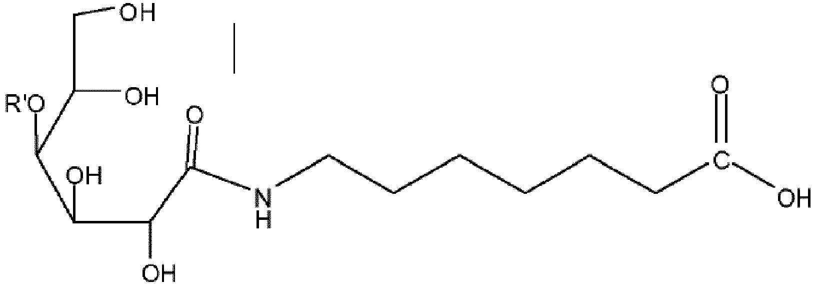 avec R' = H pour le Glu6 et avec R' = galactose pour le Lac6.

Ces dérivés sont préparés selon un art antérieur décrit dans FR 2 727 110.

Le principe actif G pourra être notamment un antihistaminique, tel que la prométhazine ou la méquitazine.

La présente invention a également pour objet un complexe d'association intermoléculaire d'un transporteur amphiphile et d'un principe actif tel que défini précédemment pour son utilisation en tant que médicament, plus particulièrement en tant qu'antihistaminique, antidépresseur, hypotenseur, neurotransmetteur, amphétamine, anesthésiant, antimigraineux, stimulant cardiaque, agoniste des récepteurs nicotiniques, vitamine ou pro-vitamine, ou anticancéreux, et notamment en tant qu'antihistaminique.

Il est décrit l'utilisation d'un complexe d'association intermoléculaire d'un transporteur amphiphile et d'un principe actif tel que défini précédemment pour la préparation d'un médicament, plus particulièrement d'un antihistaminique, d'un antidépresseur, d'un hypotenseur, d'un neurotransmetteur, d'une amphétamine, d'un anesthésiant, d'un antimigraineux, d'un stimulant cardiaque, d'un agoniste des récepteurs nicotiniques, d'une vitamine ou pro-vitamine, ou d'un anticancéreux, et notamment d'un antihistaminique.

La présente demande de brevet a également pour objet une composition pharmaceutique comprenant au moins un complexe d'association intermoléculaire d'un transporteur amphiphile et d'un principe actif tel que défini précédemment.

Cette composition pharmaceutique pourra être plus particulièrement destinée à une application topique ou transcutanée. Il pourra ainsi s'agir d'un hydrogel ou d'une émulsion.

| | |
|---|---|
| **Glu6M** | 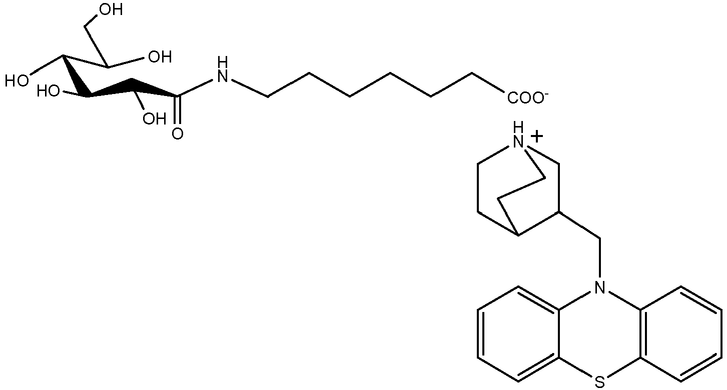 |

La présente invention a également pour objet un complexe d'association intermoléculaire d'un transporteur amphiphile et d'un principe actif tel que défini précédemment pour son utilisation en tant que médicament, plus particulièrement en tant qu'antihistaminique, antidépresseur, hypotenseur, neurotransmetteur, amphétamine, anesthésiant, antimigraineux, stimulant cardiaque, agoniste des récepteurs nicotiniques, vitamine ou pro-vitamine, ou anticancéreux, et notamment en tant qu'antihistaminique.

L'invention concerne également l'utilisation d'un complexe d'association intermoléculaire d'un transporteur amphiphile et d'un principe actif tel que défini précédemment pour la préparation d'un médicament, plus particulièrement d'un antihistaminique, d'un antidépresseur, d'un hypotenseur, d'un neurotransmetteur, d'une amphétamine, d'un anesthésiant, d'un antimigraineux, d'un stimulant cardiaque, d'un agoniste des récepteurs nicotiniques, d'une vitamine ou pro-vitamine, ou d'un anticancéreux, et notamment d'un antihistaminique.

La présente demande de brevet a également pour objet une composition pharmaceutique comprenant au moins un complexe d'association intermoléculaire d'un transporteur amphiphile et d'un principe actif tel que défini précédemment.

Cette composition pharmaceutique pourra être plus particulièrement destinée à une application topique ou transcutanée. Il pourra ainsi s'agir d'un hydrogel ou d'une émulsion.

La présente invention a également pour objet l'utilisation d'un complexe d'association intermoléculaire d'un transporteur amphiphile et d'un principe actif tel que défini ci-dessus, pour protéger et/ou pour solubiliser et/ou pour véhiculer vers son site d'action le principe actif G.

La présente invention a également pour objet un procédé de préparation d'un complexe d'association intermoléculaire d'un transporteur amphiphile et d'un principe actif tel que défini précédemment, comprenant les étapes successives suivantes :
- mélange du transporteur amphiphile de formule (II) tel que défini précédemment et du principe actif G tel que défini ci-dessus, et
- séparation du milieu réactionnel du complexe d'association intermoléculaire du transporteur amphiphile et du principe actif ainsi obtenu.

Ainsi, le complexe d'association intermoléculaire est formé par simple mise en contact de la molécule amphiphile sous sa forme acide avec le principe actif sous sa forme basique, notamment dans l'eau ou dans un autre solvant tel que le méthanol. Le complexe d'association est donc obtenu par une réaction acide/base entre la forme acide du transporteur amphiphile et la forme basique du principe actif.

Par ailleurs, il est à noter que des structures supramoléculaires, telles que des vésicules ou des micelles, se forment spontanément lors du mélange du transporteur et du principe actif en solution aqueuse, structures qui sont stables thermodynamiquement.

Le transporteur amphiphile et le principe actif seront notamment mélangés dans des proportions stoechiométriques, c'est-à-dire que l'on mélange le même nombre de moles du transporteur amphiphile et du principe actif. Par ailleurs, ce mélange peut être effectué en chauffant à une température comprise entre l'ambiante et la température d'ébullition du solvant à pression atmosphérique, et sur une durée de 1 à 72 heures.

Le complexe d'association intermoléculaire ainsi obtenu pourra alors être séparé du milieu réactionnel par filtration, et ensuite éventuellement être lyophilisé.

La présente invention sera mieux comprise à la lumière des exemples et figures non limitatifs qui suivent.

### FIGURES

La **Figure 1** représente les quantités cumulées de prométhazine qui ont diffusées à travers la peau de porc, au bout de 24h, pour une formulation A contenant de la prométhazine libre et une formulation B contenant l'association Lac6P.
La **Figure 2** représente le pourcentage de prométhazine non dégradée restant en solution, en fonction du temps, pour une solution contenant du chlorhydrate de prométhazine et une solution contenant une association Lac6P

### EXEMPLES

### Exemple 1 : Association entre l'acide 1,7-lactobionamidoheptanoïque L6 et la prométhazine (Lac6P)

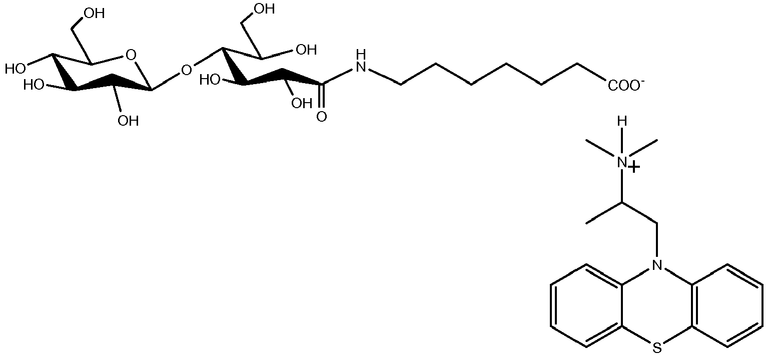

A une solution de 320 mg (0,66 mmol) de tensioactif dérivé de sucre **Lac6** dans 30 mL d'eau ultra pure, sont ajoutés 187 mg (0,66 mmol) de prométhazine. Le ballon est enveloppé de papier opaque noir, pour protéger la prométhazine, photosensible, de la lumière. Le mélange réactionnel est ensuite placé sous agitation à 25°C pendant 24h. On obtient alors une solution homogène qui, après lyophilisation, conduit de façon quantitative au produit, sous forme d'une poudre blanche.

L'association constitue une nouvelle espèce amphiphile qui forme des vésicules dans l'eau dont le diamètre hydrodynamique moyen est de 290 nm (Indice de Polydispersité (IP) = 0,12) à partir d'une CAC (Concentration d'Agrégation Critique) de 3,3.10⁻²M.

### Exemple 2 : Association entre l'acide 1,7-gluconamidoheptanoïque G6 et la prométhazine (Glu6P)

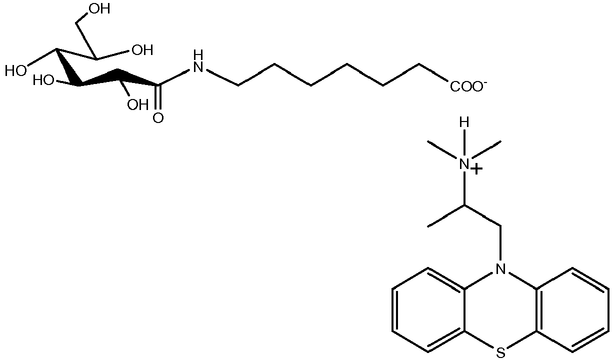

A une solution de 213 mg (0,66 mmol) de tensioactif dérivé de sucre **Glu6** dans 30mL d'eau ultra pure, sont ajoutés 187 mg (0,66 mmol) de prométhazine. Le ballon est enveloppé de papier opaque noir, pour protéger la prométhazine, photosensible, de la lumière. Le mélange réactionnel est ensuite placé sous agitation à 25°C pendant 24h. On obtient alors une solution homogène qui, après lyophilisation, conduit de façon quantitative au produit, sous forme d'une poudre blanche.

L'association constitue une nouvelle espèce amphiphile qui forme des vésicules dans l'eau dont le diamètre hydrodynamique moyen est de 285 nm (IP = 0,10) à partir d'une CAC de 1,1.10⁻²M.

### Exemple 3 : Association entre l'acide 1,7-lactobionamidoheptanoïque L6 et la méquitazine (Lac6M)

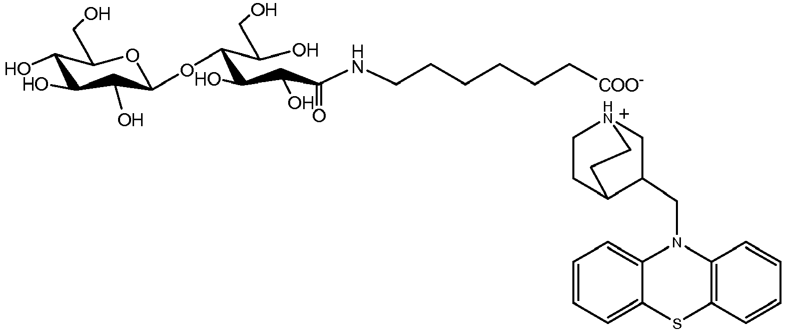

A une solution de 320 mg (0,66 mmol) de tensioactif dérivé de sucre **Lac6** dans 30mL d'eau ultra pure, sont ajoutés 214 mg (0,66 mmol) de méquitazine. Le ballon est enveloppé de papier opaque noir, pour protéger la méquitazine, photosensible, de la lumière. Le mélange réactionnel est ensuite placé sous agitation à 25°C pendant 24h. On obtient alors une solution homogène qui, après lyophilisation, conduit de façon quantitative au produit, sous forme d'une poudre blanche.

L'association constitue une nouvelle espèce amphiphile qui forme des micelles dans l'eau à partir d'une CMC (Concentration Micellaire Critique) de 3,3.10⁻²M.

### Exemple 4 : Association entre l'acide 1,7-gluconamidoheptanoique G6 et la méquitazine (Glu6M)

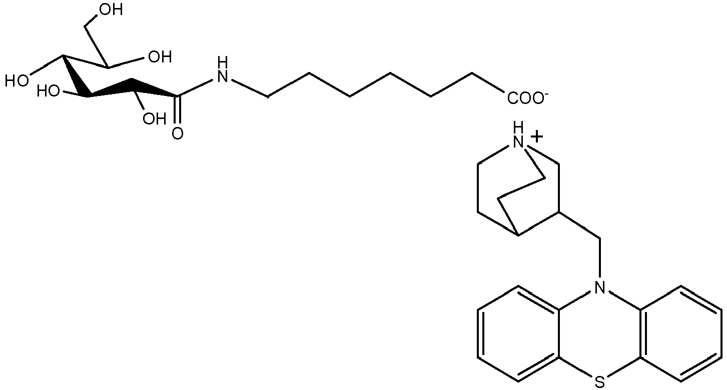

A une solution de 213 mg (0,66 mmol) de tensioactif dérivé de sucre **Glu6** dans 30mL d'eau ultra pure, sont ajoutés 214 mg (0,66 mmol) de méquitazine. Le ballon est enveloppé de papier opaque noir, pour protéger la méquitazine, photosensible, de la lumière. Le mélange réactionnel est ensuite placé sous agitation à 25°C pendant 24h. On obtient alors une solution homogène qui, après lyophilisation, conduit de façon quantitative au produit, sous forme d'une poudre blanche.

L'association constitue une nouvelle espèce amphiphile qui forme des micelles dans l'eau à partir d'une CMC de 1,1.10⁻²M.

### Exemple 5 : Etude de pénétration transcutanée menée sur deux associations réalisées avec la prométhazine et le Lac6P.

### • Formulation des échantillons

Les hydrogels étudiés sont préparés en ajoutant à un gel aqueux à 2% massique de Natrosol®, le même volume d'une solution aqueuse vésiculaire (8.10⁻⁴M soit 2% massique en principe actif) de **Lac6P** ou d'une solution aqueuse de chlorhydrate de prométhazine également à 2% massique. Nous obtenons ainsi, une formulation à 1% massique en Natrosol® ainsi qu'en principe actif.

Les différentes formules testées dans cette étude sont les suivantes :
- **formule A :** préparation de la prométhazine à 1% dans un hydrogel à 1% en Natrosol®, et
- **formule B** : préparation de l'association **Lac6P** à 1% dans un hydrogel à 1% en Natrosol®.

### • Evaluation de la pénétration cutanée

La pénétration cutanée des différentes formules a été évaluée sur des cellules de diffusion de Franz à flux dynamique de 1 cm². L'étude a été menée *ex vivo* sur de la peau d'oreille de porc en dose finie sur une durée de 24 heures.

La peau est prélevée sur des oreilles de porc fournies par les abattoirs de Montauban. Après nettoyage et tonte des oreilles, la peau est prélevée sur la face externe de l'oreille et stockée à -20°C. Pour la préparation de la membrane, la peau d'oreille de porc est décongelée la veille de l'étude. Elle est ensuite dermatomée à 500 µm à l'aide d'un dermatome afin d'obtenir une épaisseur homogène. Enfin, la peau dermatomée est découpée en échantillons carrés de 1,5 cm de côté. Ces derniers sont placés sur les cellules de diffusion, le derme en contact avec le milieu récepteur. La surface de la peau est thermostatée à une température de 32°C par un flux d'eau chauffée à 37°C.

Le milieu récepteur est composé d'une solution de NaCl à 0,9% et de BSA (albumine de sérum bovin) à 3%. Il est perfusé régulièrement à un flux de 1,5 mL/heure.

La formulation à tester est appliquée à la surface d'un échantillon de peau qui sépare le compartiment donneur du compartiment récepteur de la cellule de diffusion. Les substances sont déposées par double pesée à l'aide d'une seringue de 1 mL pour les produits visqueux. L'étude étant réalisée en dose finie, la quantité déposée est de 5 mg de formulation. Chaque formulation est testée sur deux cellules différentes tandis qu'une cellule est conservée intacte pour servir de blanc.

La formulation reste en contact avec la peau pendant 24 heures et le milieu récepteur est prélevé aux temps 2, 4, 8, 12, 18 et 24 heures pour être analysé. En fin d'étude, les volumes collectés sont mesurés. La quantité d'antihistaminique dans les différentes fractions est déterminée par un dosage par chromatographie liquide haute performance (HPLC).

Cette étude a été réalisée à 25°C sur un chromatographe Water 2695, équipé de pompes Millipore 510, d'une colonne XBridge C18 (2,1 x 150 nm, 3,5 µm). Les principes actifs sont détectés par spectrophotométrie UV-visible, à une longueur d'onde de 251 nm, avec un appareil Water 2487 Dual λ absorbance detector. L'élution est réalisée en mode gradient, avec un débit de 0,35 mL.min⁻¹. Les volumes injectés sont de 10 µL. Tous les échantillons ont été injectés trois fois.

Les conditions opératoires du gradient retenu sont les suivantes :
t₀ : 94% H₂O 0,1% HCOOH/ 6% acétontrile
t₀ à 15 min : gradient 50% H₂O 0,1% HCOOH/ 50% acétontrile
15 min à 16 min : gradient 40% H₂O 0,1% HCOOH/ 60% acétontrile

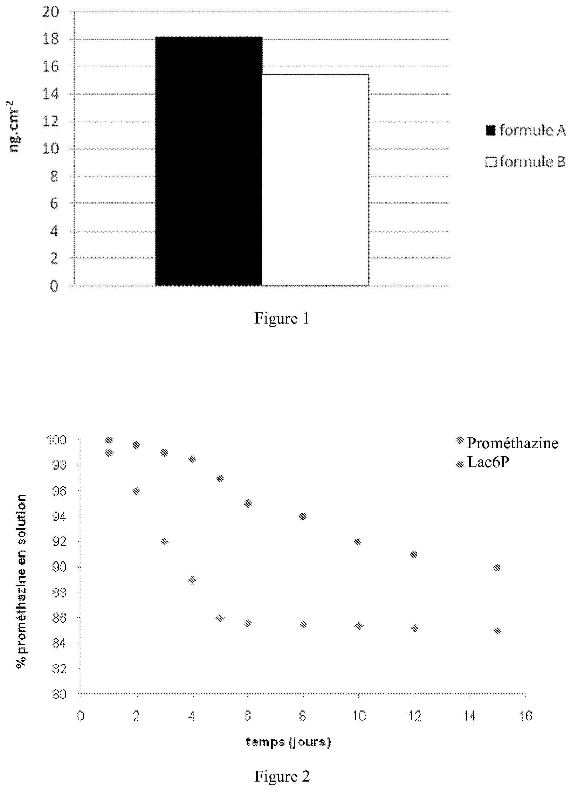

Les quantités cumulées de prométhazine mesurées par HPLC au bout de 24 heures sont représentées sur la Figure 1.

La figure 1 montre donc que la prométhazine sous forme de complexe catanionique Lac6P diffuse bien à travers la peau montrant ainsi que ce système de délivrance, vraisemblablement moins irritant est adaptée à une application topique.

### Exemple 6 : Etude de la photostabilité de Lac6P et Lac6M en comparaison respectivement avec le chlorhydrate de prométhazine et de méquitazine.

La photostabilité du principe actif est un critère important lorsqu'une administration cutanée est envisagée. En effet, la présence d'une substance photoinstable (tel que la prométhazine ou la méquitazine) au sein de l'épiderme, associée à une exposition solaire peut se traduire par une perte d'activité et une phototoxicité cutanée.

L'étude des propriétés photophysiques du chlorhydrate de prométhazine et de l'association **Lac6P** d'une part, et du chlorhydrate de méquitazine et de l'association **Lac6M,** d'autre part, a donc été menée par spectrophotométrie UV.

Cette étude a été réalisée sur un spectrophotomètre à barrette de diode Hewlett-Packart 8452A, opérant pour des longueurs d'onde comprises entre 190 et 820 nm.

Les solutions ont été analysées dans des cuves en quartz, pour des concentrations de 4.10⁻² M et de 6,6.10⁻⁵ M.

Le suivi de la photodégradation de la prométhazine a été réalisée par HPLC sur un chromatographe Water 2695, équipé de pompes Millipore 510, d'une colonne XBridge RP18 (2,1 x 100 nm, 3,5 µm). La prométhazine est détectée par spectrophotométrie UV-visible, à une longueur d'onde de 251 nm, avec un appareil Water 2487 Dual λ absorbance detector. La phase mobile est composée d'un mélange d'eau acidifiée à 0,1 % d'acide formique et d'acétonitrile 22/78. L'élution est réalisée en mode isocratique, avec un débit de 0,35 mL.min⁻¹. Les volumes injectés sont de 10 µL. Tous les échantillons ont été injectés trois fois.

Dans le cas de la méquitazine, la phase mobile est constituée d'un mélange d'eau acidifiée à 0,1 % d'acide formique et d'acétonitrile 20/70.

Une courbe de calibration est réalisée au préalable avec le chlorhydrate de prométhazine pour une gamme de concentrations comprises entre 10⁻⁵ M et 5.10⁻³ M.

La photostabilité des deux antihistaminiques sous forme de chlorhydrate ou d'association catanionique bioactive a donc été étudiée sous une irradiation UV-visible afin dévaluer l'influence de l'auto-agrégation sur les propriétés photochimiques des principes actifs.

Pour réaliser cette étude, les différentes solutions (4.10⁻² M) sont placées au bain marie à 25°C, sous lumière naturelle pendant plusieurs semaines. Ce mode d'irradiation, plus doux, a été préféré à l'utilisation d'une lampe au Xénon qui conduit à une dégradation trop rapide du principe actif et ne permet d'établir aucune comparaison.

Toutefois, avant toute analyse, plusieurs observations visuelles peuvent être faites :
- La solution de chlorhydrate de prométhazine se colore en rose dès 24h d'exposition et vire au bleu-violet après quelques jours. Puis, un composé insoluble apparaît après une quinzaine de jours sous lumière naturelle.
- La solution vésiculaire de **Lac6P** vire au rose après environ une semaine d'exposition, puis au bleu-violet. Après une vingtaine de jours, on remarque également l'apparition d'un produit insoluble en phase aqueuse, comme dans le cas du chlorhydrate correspondant.
- La solution de chlorhydrate de méquitazine devient rose après une dizaine de jours d'exposition, puis n'évolue plus.
- La solution micellaire de **Lac6M** reste incolore et limpide pendant plusieurs mois.

L'apparition tardive de la coloration rose de la solution de **Lac6P** semble donc indiquer que la dégradation de la prométhazine est légèrement retardée par l'auto-agrégation de la paire d'ions. En revanche, on a pu constater que la méquitazine se dégrade uniquement sous forme de chlorhydrate.

Cependant, afin de mieux appréhender ce phénomène, la photodégradation des chlorhydrates et des associations **Lac6P** et **Lac6M** a été suivie par spectrophotométrie visible. Cette technique d'analyse est particulièrement adaptée à l'étude de ce phénomène car elle permet de rendre compte des variations de couleur.

Ainsi, les différentes solutions présentent initialement une absorbance nulle dans le domaine du visible. Après 2 mois sous exposition naturelle, l'apparition d'une bande pour les chlorhydrates de prométhazine et de méquitazine, ainsi que pour l'association catanionique **Lac6P** confirme les changements de couleur observés. Il est important de noter qu'en spectrophotométrie visible, la couleur perçue est toujours complémentaire de la radiation absorbée. Par exemple, le chlorhydrate de méquitazine absorbe dans le vert (λ = 512 nm) et apparaît rose pourpre. L'absorbance nulle dans le domaine du visible pour l'association catanionique **Lac6M** traduit l'absence de coloration observée après 2 mois sous rayonnement UV. Les résultast obtenues sont reportés dans le tableau ci-dessous.

| | **Chlorhydrate de prométhazine** | **Chlorhydrate de méquitazine** | **Lac6P** | **Lac6M** |
|---|---|---|---|---|
| λₘₐₓ (nm) | 538 | 512 | 562 et 594 | - |
| couleur observée | violet-indigo | rose pourpre | tons de bleu | incolore |

Ces mesures montrent que la dégradation de la prométhazine est affectée par l'auto-agrégation de la paire d'ions. L'apparition tardive de la coloration rose pour la solution de **Lac6P** semble indiquer une protection durant les premiers jours d'exposition. En outre, le chlorhydrate de prométhazine présente une bande à 538 nm tandis que l'association **Lac6P** est caractérisée par 2 bandes à 562 et 594 nm (voir tableau précédent). Ces différences semblent mettre en évidence un taux d'avancement différent de la dégradation des deux composés.

Le confinement du principe actif au sein de la membrane des vésicules catanioniques semble donc modifier la dégradation de la prométhazine et de la méquitazine induite par les radiations UV, ces principes actifs se trouvant ainsi protégés.

Cependant, l'absence de coloration ne permet pas de conclure sur l'absence de dégradation. L'association de la méquitazine avec le transporteur amphiphile peut effectivement modifier la voie de dégradation du principe actif.

Afin d'affiner les résultats obtenus, la dégradation du chlorhydrate de prométhazine et de l'association catanionique **Lac6P** a été quantifiée par chromatographie liquide haute performance (HPLC). Le suivi de la dégradation est réalisé par le dosage de la quantité de principe actif non dégradé présente en solution (Figure 2).

On observe ainsi que l'auto-association de la prométhazine sous forme de vésicules catanioniques minimise la dégradation du principe actif pendant les premiers jours d'exposition (voir Figure 2). Après 5 jours sous lumière naturelle, l'association **Lac6P** et le chlorhydrate de prométhazine présentent respectivement un taux de dégradation de 3% et 13%. La cinétique de dégradation est donc retardée par l'auto-organisation de la paire d'ions. L'incorporation de la prométhazine au sein de la membrane des vésicules catanioniques se traduit par une protection significative de l'antihistaminique pendant les premiers jours d'exposition.

En outre, il a été observé que l'association catanionique **Lac6M** présente après 2 mois d'exposition un spectre UV similaire à celui du chlorhydrate de méquitazine non dégradé. Ce résultat conforte les observations réalisées en spectrophotométrie visible. L'association de la méquitazine avec le transporteur amphiphile Lac6 permet bien de protéger, sur le long terme, le principe actif vis-à-vis du rayonnement UV.

L'ensemble de ces résultats met donc en évidence des différences entre la dégradation des principes actifs sous formes libres ou associées. Dans le cas de la prométhazine, la cinétique de dégradation est retardée par l'auto-agrégation qui permet une protection du principe actif pendant les premiers jours d'exposition, tandis que dans le cas de la méquitazine, la formation de micelles par l'association catanionique **Lac6M** se traduit par une protection importante de la méquitazine durant plusieurs mois.

Ainsi, quel que soit le type d'agrégats formés par les assemblages catanioniques bioactifs en solution aqueuse, l'association entre le tensioactif dérivé de sucre et le principe actif assure la stabilité chimique de ce dernier. Ce phénomène de protection est particulièrement intéressant pour le développement de nouvelles formulations à visée dermo-cosmétique.

## Revendications

1. Complexe d'association intermoléculaire d'un transporteur amphiphile et d'un principe actif
**Caractérisé en ce que** ledit complexe est choisi parmi :
| | |
|---|---|
| **Lac6P** | 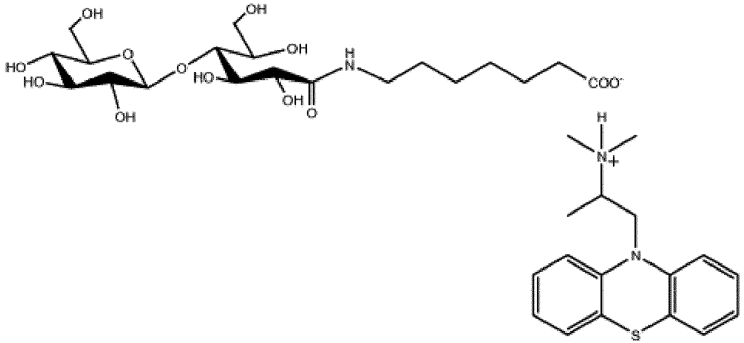 |
| **Glu6P** | 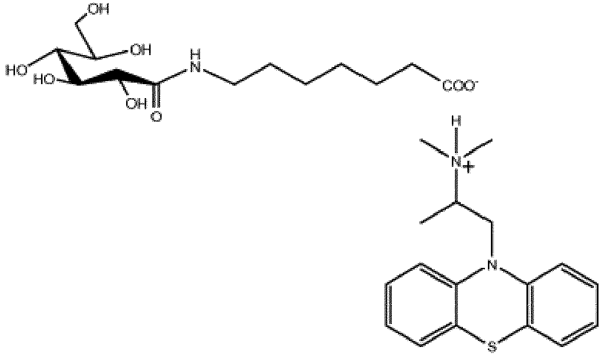 |
| **Lac6M** | 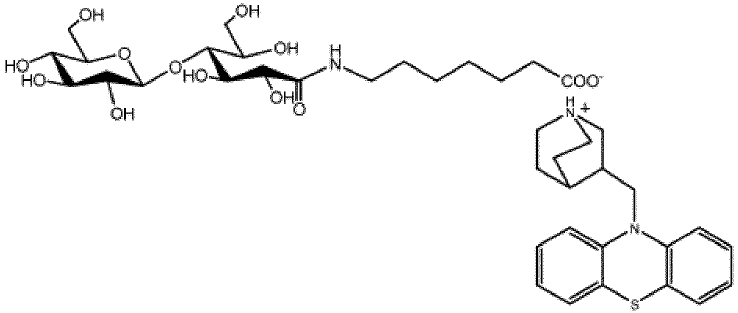 |
| **Glu6M** | 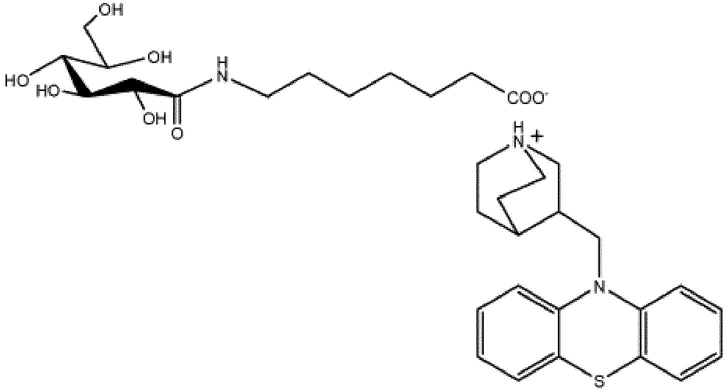 |

2. Complexe d'association intermoléculaire d'un transporteur amphiphile et d'un principe actif selon la revendication 1, pour son utilisation en tant que médicament, et notamment en tant qu'antihistaminique.

3. Composition pharmaceutique comprenant au moins un complexe d'association intermoléculaire d'un transporteur amphiphile et d'un principe actif selon la revendication 1.

4. Composition pharmaceutique selon la revendication 3, **caractérisée en ce qu'**elle est destinée à une application topique ou transcutanée.

5. Composition pharmaceutique selon la revendication 4, **caractérisée en ce qu'**il s'agit d'un hydrogel ou d'une émulsion.

6. Utilisation d'un complexe d'association intermoléculaire d'un transporteur amphiphile et d'un principe actif selon la revendication 1, pour protéger et/ou pour solubiliser et/ou véhiculer vers son site d'action le principe actif tel que défini à la revendication 1.

7. Procédé de préparation d'un complexe d'association intermoléculaire d'un transporteur amphiphile et d'un principe actif selon la revendication 1, comprenant les étapes successives suivantes :
- mélange du transporteur amphiphile de formule (II) suivante : 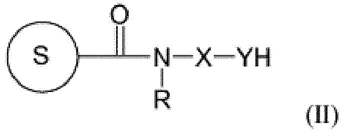 pour laquelle S, R, X, n et Y sont tels que définis à la revendication 1, et du principe actif G tel que défini à la revendication 1, et
- séparation du milieu réactionnel du complexe d'association intermoléculaire du transporteur amphiphile et du principe actif ainsi obtenu.

8. Procédé selon la revendication 7, **caractérisé en ce que** le transporteur amphiphile et le principe actif sont mélangés dans des proportions essentiellement stoechiométriques.

9. Procédé selon la revendication 7 ou 8, **caractérisé en ce que** le complexe d'association intermoléculaire du transporteur amphiphile et du principe actif est séparé du milieu réactionnel par filtration, et est ensuite éventuellement lyophilisé.

## Patentansprüche

1. Intermolekularer Assoziationskomplex eines amphiphilen Übermittlers und eines Wirkstoffs, **dadurch gekennzeichnet, dass** der Komplex gewählt ist aus:
| | |
|---|---|
| **Lac6P** | 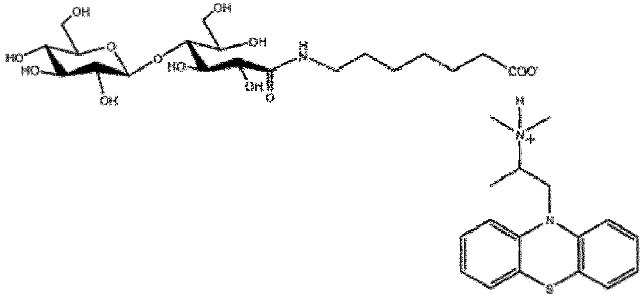 |
| **Glu6P** | 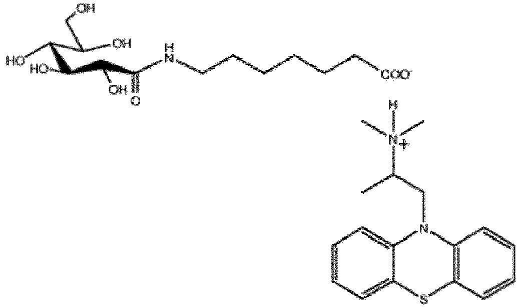 |
| **Lac6M** | 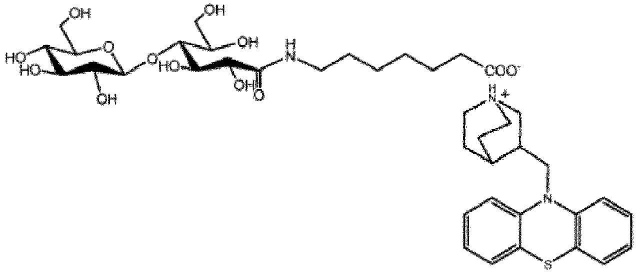 |
| **Glu6M** | 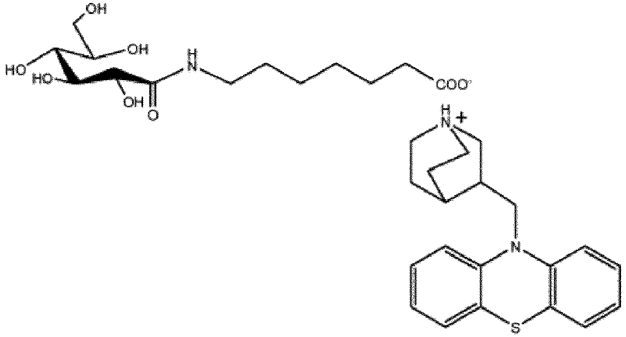 |

2. Intermolekularer Assoziationskomplex eines amphiphilen Übermittlers und eines Wirkstoffs nach Anspruch 1 zur Verwendung als Arzneimittel, insbesondere als Antihistaminikum.

3. Pharmazeutische Zusammensetzung, umfassend mindestens einen intermolekularen Assoziationskomplex eines amphiphilen Übermittlers und eines Wirkstoffs nach Anspruch 1.

4. Pharmazeutische Zusammensetzung nach Anspruch 3, **dadurch gekennzeichnet, dass** sie für eine topische oder transkutane Anwendung bestimmt ist.

5. Pharmazeutische Zusammensetzung nach Anspruch 4, **dadurch gekennzeichnet, dass** es sich dabei um ein Hydrogel oder eine Emulsion handelt.

6. Verwendung eines intermolekularen Assoziationskomplexes eines amphiphilen Übermittlers und eines Wirkstoffs nach Anspruch 1 zum Schutz und/oder zur Solubilisierung und/oder zur Beförderung des Wirkstoffs nach Anspruch 1 zu seinem Wirkort.

7. Verfahren zur Herstellung eines intermolekularen Assoziationskomplexes eines amphiphilen Übermittlers und eines Wirkstoffs nach Anspruch 1, umfassend die nachfolgenden Schritte in der nachfolgend aufgeführten Reihenfolge:
- Mischen des amphiphilen Übermittlers der nachfolgenden Formel (II): 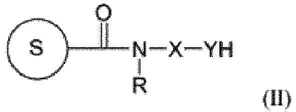 wobei S, R, X, N und Y nach Anspruch 1 definiert sind, mit dem Wirkstoff G nach Anspruch 1 und
- Separieren des Reaktionsmediums vom derart erhaltenen intermolekularen Assoziationskomplex des amphiphilen Übermittlers und des Wirkstoffs.

8. Verfahren nach Anspruch 7, **dadurch gekennzeichnet, dass** der amphiphile Übermittler und der Wirkstoff in im Wesentlichen stöchiometrischen Verhältnissen vermischt werden.

9. Verfahren nach Anspruch 7 oder 8, **dadurch gekennzeichnet, dass** der intermolekulare Assoziationskomplex des amphiphilen Übermittlers und des Wirkstoffs durch Filtration vom Reaktionsmedium separiert und anschließend evtl. gefriergetrocknet wird.

## Claims

1. Intermolecular association complex of an amphiphilic transporter and an active ingredient,
**characterized in that** said complex is chosen from:
| | |
|---|---|
| **Lac6P** | 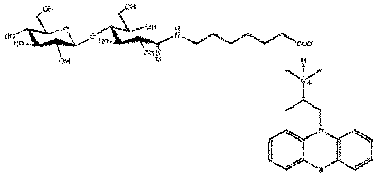 |
| **Glu6P** | 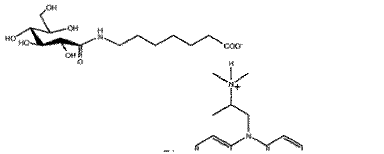 |
| **Lac6M** | 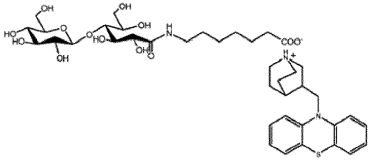 |
| **Glu6M** | 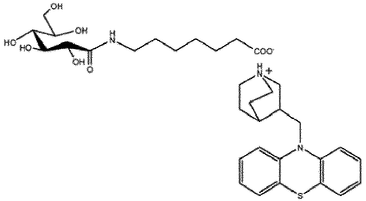 |

2. Intermolecular association complex of an amphiphilic transporter and an active ingredient according to claim 1 for its use as a medicament, and especially as an antihistamine.

3. Pharmaceutical composition comprising at least one intermolecular association complex of an amphiphilic transporter and an active ingredient according to claim 1.

4. Pharmaceutical composition according to claim 3, **characterized in that** it is intended for topical or transcutaneous application.

5. Pharmaceutical composition according to claim 4, **characterized in that** it is a hydrogel or an emulsion.

6. Use of an intermolecular association complex of an amphiphilic transporter and an active ingredient according to claim 1, to protect and/or solubilize and/or transport the active ingredient as defined in claim 1 to its site of action.

7. Method for preparing an intermolecular association complex of an amphiphilic transporter and an active ingredient according to claim 1, comprising the following successive steps:
- mixing of the amphiphilic transporter of formula (II) below: 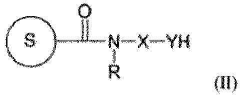 wherein S, R, X, N and Y are as defined in claim 1 and the active ingredient G is as defined in claim 1, and
- separation of the reaction medium from the intermolecular association complex of the amphiphilic transporter and the active principle thus obtained.

8. Method according to claim 7, **characterized in that** the amphiphilic carrier and the active ingredient are mixed in substantially stoichiometric proportions.

9. Method according to claim 7 or 8, **characterized in that** the intermolecular association complex of the amphiphilic transporter and the active ingredient is separated from the reaction medium by filtration, and is then optionally lyophilized.
